# EUROPEAN PATENT APPLICATION

(11) **EP 1 051 969 A1**
(43) Date of publication of application: **15.11.2000**
(21) Application number: 99303676.3
(22) Date of filing: 11.05.1999
(51) Int. Cl.: A61K 9/16, A61K 9/00

(54) **Coated antibiotic granules**

(71) Applicant: Industria Quimica E Farmaceutica Schering-Plough S.A., 22775-111 Rio de Janeiro (BR)
(72) Inventor: Souza, Emidio, Freguesia, Jacarepagua, Rio de janeiro (BR); Nunes, Jadir, Barra de Tijuca, Rio de Janeiro (BR); Martins Soares, Angela, Petropolis, Rio de Janeiro (BR)
(74) Representative: Adams, Nicola

(57) **Abstract**

The present invention refers to a process for preparation of an oral pharmaceutical composition comprising an antibiotic in a powder form comprising the steps of mixing one or more excipients comprising agglutinants, thickeners or moisteners, together with at least a film-forming compound, drying and grinding the resulting mass characterized by the resulting granules have a size ranging between 0.150 and 0.355 mm

In another embodiment, the invention refers to oral pharmaceutical compositions comprising an antibiotic in the form of a granulated-coated powder.

## Description

### Field of the Invention

The present invention refers to the preparation of an oral pharmaceutical composition, especially a pharmaceutical composition containing an antibiotic compound, which comprises masking the taste of the active substance.

### Background of the Invention

Many medicaments for oral use, mainly antibiotic medicaments, are formulated as dispersible tablets, which are products usually prepared from water-soluble raw materials that provide a rapid dissolution of the tablet when in contact with water. This type of formulation is employed, for instance, for antibiotic medicaments for children, which are easily dissolved in a spoonful of water for later administration to the child.

However, such medicaments usually have an unpleasant bitter taste, which impairs the fulfillment of an adequate dosage especially in the case of children.

Physiologically, the taste is a sensorial response resulting from chemical stimulation in the taste buds located in the tongue, determining four different tastes, namely salty, acid, sweet and bitter. Usually these sensations are linked to the capacity of ionizing the substances in solution, but various organic components and active drugs such as antibiotics stimulate a bitter response, even if they are not capable of being ionized in aqueous means. In addition, another important effect observed with respect to these compounds is a response of residual bitter taste after ingestion thereof, which is also known as "aftertaste", thus impairing the acceptance, by the patients, of the products that contain these drugs.

Various substances have been used in the medicament industry for the scope of masking the unpleasant taste of solid oral pharmaceutical compositions.

Compounds such as polyvinylpyrrolidone, for example, are widely utilized in the pharmaceutical industrial processes for this scope and, specifically in the case of polyvinylpyrrolidone, it has become a quite versatile polymeric thickener. It is an inert compound with the advantage of being soluble in both water and ethyl alcohol at a concentration usually ranging from 3 to 15%.

Another group of compounds that are much used in processes of masking the taste of solid oral formulations include the cellulose esters. Ethylcellulose, for example, is widely used to formulate coating films for prolonged release of drugs and also for coatings with organic solvents, in association with other cellulose polymers such as hydroxypropylcellulose, optimizing the formed films. Tablets coated with ethylcellulose become easier to handle and process, have better covering, and in addition this compound has not odor or taste and is highly stable as regards both the storing conditions, namely light and heat, and the physiological aspects of compatibility with the human or animal organism, being used in alcoholic solutions, usually at concentrations of about 0 5 - 2.0% as a coating agent.

US Pat 5,082,669 describes a particulate oral pharmaceutical preparation, easy to release, prepared by a process in which nucleus-components of a compound of bad taste, in granulate state, are dispersed in a liquid and fed to a mixer-granulator, and a suspension containing taste-masking compounds (for example, ethylcellulose and polyvinylpyrrolidone) is sprayed over the nucleus composition and then dried. The technique, however, allows one to prepare only tablets containing a taste-masking film, and does not make it possible to prepare the composition in the form of a powder suitable for being used later for the preparation of a suspension.

### Objective of the Invention

An objective of the present invention is to provide a process for preparing an oral pharmaceutical composition containing antibiotic in the form of an extemporaneous powder, which permits one to mask the bad taste of an active principle and also to mix it later with a diluent to form a suspension.

### Summary of the Invention

The present invention provides a process for preparing an oral pharmaceutical composition comprising an antibiotic in the form of a powder, which comprises the steps of mixing, preferably under stirring, the antibiotic active principle with one or more excipients comprising agglutinants, thickeners or moisteners, together with at least one film-forming compound drying and grinding the resulting mass so as to form granules with sizes ranging between 0.150 and 0.355 mm

In another aspect, the invention provides an oral pharmaceutical composition comprising an antibiotic in the form of an agglutinated and coated powder having granule sizes ranging from 0.150 to 0.355 mm

In a further aspect of the present invention, an oral pharmaceutical composition is provided, in the form of a suspension, comprising an antibiotic in the form of an agglutinated and coated powder having granule sizes ranging from 0.150 to 0.355 mm, mixed with an antibiotic compatible pharmaceutically acceptable carrier

According to another embodiment, the invention provides a kit that comprises at least one compartment containing an antibiotic component in the form of an agglutinated and coated powder having granule sizes ranging from 0.150 to 0.355 mm and at least one more compartment comprising an antibiotic compatible pharmaceutically acceptable diluent

### Detailed Description of the Invention

After detailing the studies, the inventors find that antibiotics, which are compounds usually having a bitter and bad taste, can be formulated as an agglutinated and coated powder with the bitter taste masked and that permits its later dilution with an appropriate liquid to form a suspension. Thus, it has now become possible to prepare liquid oral antibiotic compositions with masked taste, which renders the consumption thereof pleasant, especially for administration to children.

The wet granulation process used in the present invention comprises adding liquids to a powder within an adequate container, provided with a stirring system, with a view to producing an easy-to-handle agglomerate or granule, changing the ingredients into powders having an adequate fluidity, increasing the density and the particle size of the active principles, reducing the formation of powder in the productive system. This method has the advantage of permitting one to process different types of materials together, in a single equipment and in a single batch, altering the physical characteristics of the components until an ideal mixture is reached in accordance with the scope of the process.

In the process called "granulation-coating" according to the present invention, the antibiotic particles are agglomerated by adding one or more agglutinating, thickening or moistening compounds. These compounds are preferably chosen from the group comprising hydrophilic gums and sugars, starches, polymers, jelly and cellulose derivatives which may be hydrophilic and mixtures thereof, which have the capacity of providing a consistent mixture, when the powder is moistened by water or another adequate solvent, forming a mass that can be subdivided, dried and ground. Among the preferred compounds for the scope of the invention, cellulose, lactose, saccharose, and polyvinylpyrrolidone can be cited. Polyvinylpyrrolidone is particularly indicated for obtaining granules resistant to the standardization process, generating a great percentage of granules within the specified range of from 0.150 to about 0.355 mm and with a low generation of powder (< 0.15 mm). The resistance imparted to the granule is also of great interest as far as its preservation is concerned, since the harder it is the less it will be liable to fragmenting. However, the hardness may not be excessive, which would alter the good characteristics of the granulate and increase its period of disintegration.

The composition formed in the mixing step is normally dried or allowed to dry before grinding. The drying process need not involve heating (although it may do so), and need not remove all liquid present, but is at least sufficient to allow grinding of the composition to form granules having a size in the range 0.150 to 0.355 mm. If the composition can be ground to the desired size without drying then the drying step may be omitted, if desired.

It has now been surprisingly found that a standardization of the size of grains resulting from the grinding of the mass obtained in accordance with the above mixture within a range of from 0.150 to about 0.355 mm allows one to obtain an ideal extemporaneous powder to be coated with a taste-masking compound, thus permitting the formation of a final powdered antibiotic product that can be formulated into a suspension. This standardized range for the grains size further ensures that there will not be intolerance to the masking of the granules by the patient or even a retention of the granules in the oral cavity.

The step of "granulation-coating" of the process of the invention includes, therefore, subsequently mixing the composition resulting from the addition of the antibiotic with the thickening, agglutinating or moistening compounds with a film-forming compound that is saliva insoluble in order to mask the bitter taste, but that does not interfere with the dissolution of the active principle and its respective bioavailability and activity in the organism. As film-forming compounds, for example, cellulose ethers such as ethylcellulose, methacrylic acid based cationic or anionic nature homo or co-polymers, especially copolymers, and methacrylic acid neutral esters or polymers thereof, especially the esters, can be used. Examples of such compounds are dimethylaminocetyl methacrylate cationic copolymers and methacrylic acid neutral esters or methacrylic acid anionic polymers and methacrylic acid methyl ester.

The process of the present invention is especially useful for preparing oral pharmaceutical compositions of antibiotics such as roxytromicin, which has a particularly bitter taste.

The composition of the granulated-coated powdered antibiotic with masked taste can then be formulated as a suspension by adding said granulated-coated powder to an appropriate diluent.

The process claimed now can further include the addition of other compounds with a view to improving, for instance, the preservation charactenstics and mainly the fluidity of the powder obtained as described above. Compounds usually employed for this scopes are colloidal silicon dioxide and crospovidone. Silicon dioxide, for example, in the form in which it is widely used in the pharmaceutical industry has a surface area of about 200 m²/g, which imparts to it the characteristic of absorbing materials and moisture due to the presence of silanol and hydroxyl groups in its structure, besides imparting greater fluidity to the resultant powder. This compound is preferably used at concentrations ranging from 0.1 to 2.0% by weight.

On the other hand, crospovidone, which is a derivative from the polyvinylpyrrolidone polymerization, is in the form of a white powder, insoluble in most usual solvents, with a porous structure, having a large surface area, and being virtually tasteless and odorless. Crospovidone is also widely used in the pharmaceutical industry and, in the case of the present invention, it is used as a stabilizer of the final composition when in form of a suspension, at concentrations of use of up to about 10% by weight, depending upon the viscosity and the sedimentation time desirable for the suspension.

Another important component in the preparation of a composition according to the invention, in the form of a suspension, is the diluent that, however, may comprise carriers that are conventionally used in this kind of oral antibiotic composition. Examples of such carriers comprise sorbitol in solution or propyleneglycol. The diluent in suspension may further contain additional components such as edulcorating agents, antifoaming agents, preserving agents, flavors for imparting an even more pleasant taste to the final product, sweetening agents and other usual ingredients of this kind of formulation.

It should be pointed out that the concentrations of each ingredient in the oral pharmaceutical composition comprising an antibiotic in the form of a powder or a formulation in suspension can be determined in accordance with the objectives of each case in particular, and does not represent a determining factor for the results achieved now.

The oral composition containing a granulated-coated powdered antibiotic to be used in the form of a suspension can be prepared as composition of the ready-to-use type, as well as it can be prepared in a kit where the two main components, that is to say, the referred-to granulated-coated powdered antibiotic and the diluent are maintained in separate compartments until the moment of administration, when the two main components are then mixed to form the suspension effectively.

The illustrative examples presented below will serve to describe the present invention in a better way. However, the data and procedures illustrated merely refer to some embodiments of the present invention and should not be taken as being limitative of its scope.

### EXAMPLES

### Preparation of Antibiotics in the Form of a Coated Granulate

2,000 g of active roxytromicin is added in a stainless steel mixer Maintaining an average velocity of the mixer, 0.060 g of polyvinylpyrrolidone USP was slowly added.

Maintaining the same velocity of stirring, 0.400 g of ethylcellulose NF was added. The resultant mass was passed through a Fitzmill mill and the granulate was dried in an oven at 40° C for 8 hours.

Then the dried granulate was again passed through a Fitzmill mill with the adequate mesh for obtaining standardized grains ranging from 0.150 to 0.355 mm.

The powder obtained above was mixed with 0.200 g of colloidal NF silicon dioxide and 0.340 g of NF crospovidone, in a "V"-shaped stainless steel mixer. The stirring was maintained for about 40 minutes, the coated granulate being finally obtained.

### Preparation of a Formulation in the Form of a Suspension

For preparing 100 mL of suspension, the coated granulate obtained in Example 1 was mixed with the following diluent:
- Sodium saccharin USP 0.050 g
- Dimethylpolysiloxane NF 0.750 g
- Methylparaben NF 0.180 g
- Propylparaben NF 0.020 g
- Propyleneglycol USP 1.000 g
- Fruits flavors 0.030 g
- Purified water 0.200 g
- 70% Sorbitol solution USP qsp 97.000 mL

### 3) Preparation of a Formulation in the Form of a Suspension

100 mL of suspension was prepared as in example 2, following the same steps of preparing powder mentioned in example 1, but using the following composition:

### Active Principle:

- Active roxytromicin 2.000 g

### Excipients:

- Methacrylate based cationic copolymer 1.220 g
- Saccharose NF 0.417 g
- Lactose NF/Cellulose NF 3:1 1.667 g
- Colloidal silicon dioxide NF 0.667 g
- Crospovidone NF q.s.p. 6.500 g

### Diluents:

- Sodium saccharin USP 0.050 g
- Diimethylpolysiloxane NF 0.750 g
- Methylparaben NF 0.180 g
- Propylparaben NF 0.020 g
- Propyleneglycol USP 1.00 g
- Tuti-fruti artificial flavor 0.030 g
- Purified water 20.000 g
- Sorbitol solution 70% USP qsp 93.500 mL

Any features described or claimed herein may be used in any combination except where clearly incompatible.

The present invention provides a process for preparing an oral pharmaceutical preparation comprising mixing an antibiotic with one or more excipients selected from agglutinants, thickeners and moisteners, and optionally a solvent, and at least one film-forming substance, optionally drying the mixture, and grinding the mixture to form granules having a size in the range of 0.150 to 0.355 mm.

The invention also provides a process for preparing an oral pharmaceutical preparation comprising mixing an antibiotic with one or more excipients selected from agglutinants, thickeners and moisteners, and optionally a solvent, optionally drying the mixture, grinding the mixture to form granules having a size in the range of 0.150 to 0.355 mm, and coating the granules with at least one film forming substance.

Any of the specified steps may be carried out in one or more stages, which may be separated from each other by another step. Thus, for example, there may be two grinding stages, with a drying step between them. The specified steps may be carried out in any appropriate order, and other steps may be included in the process, if appropriate, for example, mixing the granules of the desired size with stabilizing agents and/or agents imparting fluidity.

## Claims

1. A process for preparing an oral pharmaceutical preparation comprising mixing an antibiotic with one or more excipients selected from agglutinants, thickeners and moisteners, and optionally a solvent, and at least one film-forming substance, optionally drying the mixture, and grinding the mixture to form granules having a size in the range of 0.150 to 0.355 mm.

2. A process for preparing an oral pharmaceutical composition comprising an antibiotic in the form of a powder, which comprises the steps of mixing, under stirring, the antibiotic active principle with one or more excipients comprising agglutinants, thickeners or moisteners, together with at least one film-forming compound, drying and grinding the resultant mass, the resultant granules having a size in the range of 0.150 to 0.355 mm.

3. A process according to claim 1 or claim 2 in which the antibiotic is roxithromycin.

4. A process according to any one of claims 1 to 3 in which the agglutinants, thickeners or moisteners are selected from the group comprising hydrophilic gums, sugars, starches, polymers, jelly, cellulose derivatives or mixtures thereof.

5. A process according to claim 4, in which the agglutinants, thickeners or moisteners are selected from cellulose, lactose, saccharose and polyvinylpyrrolydone.

6. A process according to any one of the preceding claims, in which said at least one film-forming compound is selected from the group comprising cellulose ethers such as ethylcellulose, methacrylic acid based cationic or anionic nature polymers and copolymers and methacrylic acid neutral esters.

7. A process according to claim 6, in which the film-forming compound is selected from ethylcellulose, dimethylaminocetylmethacrylate cationic copolymers and methacrylic acid neutral esters, or methacrylic acid anionic polymers and methacrylic acid methyl ester.

8. A process according to any one of the preceding claims, in which collodial silicon dioxide and/or crospovidone are added, advantageously both, optionally in the mixing step.

9. An oral pharmaceutical preparation comprising an antibiotic in the form of an agglutinated and coated powder, obtained in accordance with the process of any one of claims 1 to 8.

10. An oral pharmaceutical preparation in the form of a suspension comprising an antibiotic in the form of an agglutinated and coated powder obtained in accordance with any one of claims 1 to 8, and mixing an antibiotic compatible pharmaceutically acceptable diluent.

11. A pharmaceutical preparation according to claim 10, in which the diluent is selected from sorbitol in solution and propyleneglycol.

12. A kit comprising a pharmaceutical composition, the kit comprising at least one compartment containing an antibiotic component in the form of an agglutinated and coated powder obtained in accordance with the process of any one of claims 1 to 8 and at least another compartment containing an antibiotic compatible pharmaceutically acceptable diluent.

13. A kit according to claim 12, characterized in that the diluent is selected from sorbitol in solution and propyleneglycol.

14. A process for preparing an oral pharmaceutical preparation comprising mixing an antibiotic with one or more excipients selected from agglutinants, thickeners and moisteners, and optionally a solvent, optionally drying the mixture, grinding the mixture to form granules having a size in the range of 0.150 to 0.355 mm, and coating the granules with at least one film forming substance.

15. An oral pharmaceutical preparation comprising an antibiotic preparation obtainable by the process of any one of claims 1 to 8 and 14, or a suspension thereof in an antibiotic compatible pharmaceutically acceptable diluent.

16. A kit comprising a pharmaceutical preparation comprising an antibiotic preparation obtainable by the process of any one of claims 1 to 8 or 14, and an antibiotic compatible pharmaceutically acceptable diluent.

17. Use of a film-forming substance in the preparation of a granular oral antibiotic pharmaceutical preparation, preferably having a granule size in the range of 0.150 to 0.355 mm.
